# EUROPÄISCHE PATENTSCHRIFT

(11) **EP 3 372 609 B1**
(45) Veröffentlichungstag und Bekanntmachung des Hinweises auf die Patenterteilung: **28.04.2021**
(21) Anmeldenummer: 17159900.4
(22) Anmeldetag: 08.03.2017
(51) Int. Cl.: C07F 7/18

(54) **VERFAHREN ZUR HERSTELLUNG VON TRIS[3-(ALKOXYSILYL)PROPYL]ISOCYANURATEN**
PROCESS FOR THE PREPARATION OF TRIS[3-(ALKOXYSILYL)PROPYL]ISOCYANURATES
PROCÉDÉ DE PRÉPARATION DE TRIS[3-(ALKOXYSILYL)PROPYL]ISOCYANURATES

(43) Veröffentlichungstag der Anmeldung: 12.09.2018
(73) Patentinhaber: Evonik Operations GmbH, 45128 Essen (DE)
(72) Erfinder: ALBERT, Philipp, 79618 Rheinfelden (DE); JUST, Eckhard, 79618 Rheinfelden (DE)
(74) Vertreter: Evonik Patent Association

(56) Entgegenhaltungen:
- EP-A2- 1 150 346
- US-A- 3 517 001
- US-A- 5 986 124
- YOSHINO N ET AL: "Synthesis of bone formation deriving biosilanes", COLLOIDS AND SURFACES. B, BIOINTERFACES, ELSEVIER, AMSTERDAM, NL, Bd. 66, Nr. 1, 1. Oktober 2008 (2008-10-01), Seiten 71-76, XP022939862, ISSN: 0927-7765, DOI: 10.1016/J.COLSURFB.2008.05.009 [gefunden am 2008-05-24]

## Beschreibung

Die vorliegende Erfindung betrifft ein besonders wirtschaftliches Verfahren zur Herstellung von Tris[3-(Trialkoxysilyl)propyl]isocyanurat, Tris[3-(Alkyldialkoxysilyl)propyl]isocyanurat sowie Tris[3-(Dialkylalkoxysilyl)propyl]isocyanurat (nachfolgend in Summe auch kurz Tris[3-(alkoxysilyl)propyl]isocyanurate genannt), wobei man 1,3,5-Triallyl-1,3,5-triazin-2,4,6(1H,3H,5H)-trion mit einem Hydrogentrialkoxysilan, Hydrogenalkyldialkoxysilan bzw. Hydrogendialkylalkoxysilan in Gegenwart eines Pt-Ktalysators, einer Carbonsäure und einem weiteren Cokatalysator hydrosilyliert.

Tris[3-(alkoxysilyl)propyl]isocyanurat sind Silane, die als Vernetzer eingesetzt werden können. Durch die drei Alkoxysilylgruppen, von der jede Alkoxysilylgruppe nach Hydrolyse eine, zwei bzw. drei chemische Bindungen eingehen kann, sind theoretisch drei bis hin zu neun chemische Bindungen möglich. Durch diese starken Vernetzungsmöglichkeiten sind Tris[3-(alkoxysilyl)propyl]isocyanurate für verschiedene Anwendungen interessant. Ein weiterer Vorteil von Tris[3-(alkoxysilyl)propyl]isocyanuraten ist die hohe Temperaturbeständigkeit, die den Einsatz im Hochtemperaturbereich ermöglicht. Tris[3-(alkoxysilyl)propyl]isocyanurate können daher vorteilhaft als Vernetzer zum Beispiel in Lack- und Kautschukformulierungen sowie als Haftvermittler in Lacken und Klebstoffen in den verschiedensten Industrien eingesetzt werden. Durch die hohe Vernetzungsdichte können darüber hinaus kratzfeste Beschichtungen sowie Barriereschichten erzeugt werden.

JP4266400B beschreibt die Herstellung einer aromatischen Silanverbindung durch Hydrosilylierung einer aromatischen Vinylverbindung. Als Katalysator wird ein Platinkomplex in Anwesenheit einer Carbonsäure eingesetzt.

US 5,986,124 betrifft ein Verfahren zur Herstellung einer Silanverbindung durch Hydrosilylierung einer Kohlenstoffdoppelbindung mittels eines Trialkoxyhydrogensilans in Anwesenheit eines Platinkatalysators und einer Carbonsäure. Durch den Einsatz von Platinkatalysatoren mit Carbonsäuren kann bei der Hydrosilylierung zwar ein Umsatz von ca. 80 % erreicht werden, jedoch weisen so erhaltene Rohprodukte noch einen erheblichen Anteil an Verunreinigungen bzw. Nebenprodukte auf.

EP 0587462 beschreibt eine Zusammensetzung aus einem ungesättigten Polyorganosiloxan, einem Organowasserstoffpolysiloxan, einer Säure, einer Platinverbindung sowie Additiven, wobei die Komponenten in Wasser emulgiert und zur Trennschichtbehandlung eingesetzt werden. Die Vernetzung über eine Hydrosilylierung erfolgt beim Erwärmen.

EP 0856517 offenbart einen Prozess zur Hydrosilylierung einer ungesättigten Verbindung in Anwesenheit einer Metallverbindung der 8. bis 10. Nebengruppe des Periodensystems der Elemente. Die Hydrosilylierung wird in Anwesenheit eines Beschleunigers durchgeführt.

In EP 1869058 bzw. WO 2006/113182 wird ein Verfahren zur Herstellung von Tris[3-(Trialkoxysilyl)propyl]isocyanurat vorgestellt. Die Herstellung läuft über das Cracken von Silylorganocarbamat in Anwesenheit einer katalytischen Menge eines Carboxylatsalzes.

EP 0583581 lehrt die Herstellung eines Silylorganocarbamats aus einem Aminosilan. Das Silylorganocarbamat wird nachfolgend in Anwesenheit eines "Crack-Katalysators" zum Silylisocyanurat umgesetzt.

EP 1885731 offenbart ein Verfahren zur Herstellung von Isocyanatosilanen und Silylisocynaurat. Die Synthese startet mit einem Silylorganocarbamat. Durch katalytisches Cracken wird das Isocyanatosilan freigesetzt, wobei die Umsetzung des Isocyanatosilans zum Silylisocyanurat in einer Trimerisationsreaktionszone erfolgt.

CA 943544 beschreibt die Herstellung eines Silylorganoisocyanurats aus einem Halogenalkylsilan und einem Metallcyanat in Anwesenheit eines Lösungsmittels. Das Lösemittel sowie das entstehende Salz werden nach der Reaktion abgetrennt.

US 3,607,901 betrifft die Herstellung von Isocyanatosilanen und Isocyanuratosilanen ausgehend von Chloralkyltrialkoxysilanen und einem Metallcyanat.

US 3,517,001 lehrt u.a. die Herstellung von 1,3,5-Tris(trimethoxysilylpropyl)isocynanurat durch Hydrosilylierung von 1,3,5-Tris(allylisocynaurate) mit Trimethoxysilan in Anwesenheit von Hexachloroplatinsäure. Die Ausbeute wird mit 40 % angegeben.

US 3,821,218 beschreibt die Herstellung von 1,3,5-Tris(trimethoxysilylpropyl)isocyanurat ausgehend Chlorpropyltrimethoxysilansilan und Kaliumcyanat in DMF als Lösungsmittel.

US 2013/0158281 offenbart einen Prozess zur Hydrosilylierung einer ungesättigten Verbindung mit einem Silylhydrid. Als Katalysator werden Fe-, Ni-, Mn- oder Co-Komplexe verwendet.

CN 101805366 beschreibt die Herstellung von 1,3,5-Tris(trimethoxysilylpropyl)isocyanurat durch Cyclokondensation von Isocyanatopropyltrimethoxysilan.

CS 195549 betrifft die Hydrosilylierung von Vinylcyclohexan mit Hydrogensilanen. Dabei wird in Beispiel 4 Vinylcyclohexan mittels Triethoxysilan in Gegenwart von Platinsäure und Trifluoressigsäure hydrosilyliert.

Der vorliegenden Erfindung lag die Aufgabe zugrunde, ein Verfahren zur Herstellung von Tris[3-(alkoxysilyl)propyl]isocyanuraten, d.h. aus der Reihe Tris[3-(Trialkoxysilyl)propyl]isocyanurat, Tris[3-(Alkyldialkoxysilyl)propyl]isocyanurat sowieTris[3-(Dialkylalkoxysilyl)propyl]isocyanurat, wobei Alkyl insbesondere -aber nicht ausschließlich- für Methyl oder Ethyl sowie Alkoxy für Methoxy oder Ethoxy stehen, bereitzustellen, bei dem ein Pt-Katalysator in Verbindung mit einer Carbonsäure eingesetzt und man die zuvor aufgezeigten Nachteile gegebenenfalls durch eine gezielte Reaktionsführung, gezielte Einsatzstoffverhältnisse und/oder weiterer Zusätze mindert. Darüber hinaus bestanden auch die Anliegen, nach Möglichkeit das Verfahren mit einer möglichst geringen Konzentration an teurem Platin sowie ohne den gesonderten Zusatz eines aliphatischen oder aromatischen Lösemittels durchzuführen und die Ausbeute an Zielprodukt zu steigern. Ebenfalls möchte man den im Zielprodukt verbleibenden Gehalt an Carbonsäure möglichst gering halten.

Gelöst wird die Aufgabe gemäß den vorliegenden Patentansprüchen. Überraschenderweise wurde gefunden, dass wesentlich bessere Ausbeuten an Zielprodukt, d.h. einem Tris[3-(alkoxysilyl)propyl]isocyanurat, erzielt werden, wenn man bei der Durchführung der Hydrosilylierung
- in Schritt A ein Gemisch enthaltend 1,3,5-Triallyl-1,3,5-triazin-2,4,6(1H,3H,5H)-trion als Olefinkomponete, mindestens eine Carbonsäure und einen Pt-Katalysator vorlegt, das Gemisch auf eine Temperatur von 50 bis 140 °C erwärmt,
- in Schritt B unter Durchmischung mindestens ein Hydrogenalkoxysilan aus der Reihe Hydrogentrialkoxysilan, Hydrogenalkyldialkoxysilan, Hydrogendialkylalkoxysilan [kurz H-Silan(e)] genannt] dem Gemisch aus Schritt A zusetzt,
- in Schritt C das Gemisch aus Schritt B unter Durchmischung reagieren lässt, wobei man dem Gemisch als weiteren Cokatalysator mindestens eine definierte Menge an Alkohol zusetzt und
- in Schritt D das so erhaltene Produktgemisch aufarbeitet
und so die Umsetzung sowie die Selektivität und damit die Ausbeute der Hydrosilylierung deutlich fördert.

Dabei hat sich als besonders vorteilhaft erwiesen, das man das vorliegende Verfahren vorzugsweise mit einem homogen Platin(0)-Komplexkatalysator, insbesondere einem "Karstedt-Katalysator", einem "Speier-Katalysator", Hexachloroplatin(IV)säure, oder einem geträgerten, d.h. heterogen Pt-Katalysator, beispielsweise Pt auf Aktivkohle, durchführt. Darüber hinaus setzt man einen "Karstedt-Katalysator" bevorzugt als Platin(0)-Komplexkatalysator-Lösung ein, insbesondere gelöst in Xylol oder Toluol.

Ferner ermöglicht die vorliegende Verfahrensweise, den Gehalt an Pt-Katalysator bzw. Pt-Verlust zu verringern und so teures Pt einzusparen. Weiter hat sich beim vorliegenden Verfahren als besonders vorteilhaft erwiesen, eine Carbonsäure aus der Reihe Benzoesäure, 3,5-Di-tert-butylbenzoesäure, 3,5-Di-tert-butyl-4-hydroxy-benzoesäure, 2,2-Dimethylpropionsäure, Propionsäure und/oder Essigsäure einzusetzen.

Ein nach dem vorliegenden Verfahren erhaltenes Produktgemisch arbeitet man geeigneterweise durch Destillation, optional unter vermindertem Druck, auf und gewinnt das gewünschte (Ziel-)Produkt.

Bei Einsatz eines Heterogenkatalysators kann man diesen geeigneterweise vor der Destillation vom Produktgemisch trennen, beispielsweise durch Filtration oder Zentrifugieren, und kann diesen so zurückgewonnen Pt-Katalysator vorteilhaft in den Prozess recyclieren.

So gewinnt man das Zielprodukt als Sumpfprodukt bei der nach der Reaktion sowie ggf. nach der Abtrennung eines Heterogenkatalysators durchgeführten Destillation; das Zielprodukt wird bei der destillativen Aufarbeitung nicht überdestilliert und fällt als farbloses Sumpfprodukt an.

Gemäß dem vorliegenden Verfahren können so die Doppelbindungen der hier eingesetzten Olefinkomponente vorteilhaft praktisch vollständig hydrosilyliert werden und es entstehen vorteilhaft nur sehr wenige Nebenprodukte.

Darüber hinaus kann das vorliegende, d.h. erfindungsgemäße Verfahren vorteilhaft ohne einen gesonderten Zusatz eines aliphatischen oder aromatischen Kohlenwasserstoffs als Löse- bzw. Verdünnungsmittel und einem nur geringen Anteil an der (Co-)Katalysatorkomponente Carbonsäure, die im Zielprodukt verbleibt, durchgeführt werden.

Gegenstand der vorliegenden Erfindung ist somit ein Verfahren zur Herstellung eines Tris[3-(alkoxysilyl)propyl]isocyanurats aus der Reihe Tris[3-(Trialkoxysilyl)propyl]isocyanurat, Tris[3-(Alkyldialkoxysilyl)propyl]isocyanurat und Tris[3-(Dialkylalkoxysilyl)propyl]isocyanurat durch Hydrosilylierung,
indem man
- in Schritt A ein Gemisch enthaltend 1,3,5-Triallyl-1,3,5-triazin-2,4,6(1H,3H,5H)-trion als Olefinkomponete, mindestens eine Carbonsäure und einen Pt-Katalysator vorlegt, das Gemisch auf eine Temperatur von 40 bis 140 °C erwärmt,
- in Schritt B unter Durchmischung mindestens ein Hydrogenalkoxysilan aus der Reihe Hydrogentrialkoxysilan, Hydrogenalkyldialkoxysilan, Hydrogendialkylalkoxysilan [kurz H-Silan(e)] genannt] dem Gemisch aus Schritt A zusetzt,
- in Schritt C das Gemisch aus Schritt B unter Durchmischung reagieren lässt, wobei man dem Gemisch als weiteren Cokatalysator mindestens eine definierte Menge an Alkohol zusetzt und
- in Schritt D das so erhaltene Produktgemisch aufarbeitet.

Beim erfindungsgemäßen Verfahren setzt man H-Silan zu Olefinkomponente vorteilhaft in einem molaren Verhältnis von 1 zu 0,1 bis 1, vorzugsweise von 1 zu 0,2 bis 0,4 ein, insbesondere - um hier stellvertretend und beispielhaft nur einige der möglichen und für den Fachmann aus den vorangehenden bzw. vorliegenden Angaben ersichtlichen bzw. erschließbaren Zwischenwerte zu nennen - 1 : 0,13, 1 : 0,15, 1 : 0,18, 1 : 0,23, 1 : 0,25, 1 : 0,28, 1 : 0,3, 1 : 0,33, 1 : 0,35, 1 : 0,38.

Dabei setzt man als H-Silan bevorzugt Hydrogentrimethoxysilan (TMOS), Hydrogentriethoxysilan (TEOS), Methyldiethoxysilan (DEMS), Methyldimethoxysilan (DMMS), Dimethylethoxysilan (DMES) und/oder Dimethylmethoxysilan (MDMS) ein.

Ferner wird beim erfindungsgemäßen Verfahren als Olefinkomponente 1,3,5-Triallyl-1,3,5-triazin-2,4,6(1H,3H,5H)-trion eingesetzt.

Vorteilhaft bevorzugt man beim erfindungsgemäßen Verfahren den Einsatz von H-Silan zu Alkohol in einem molaren Verhältnis von 1 zu 0,005 bis 0,3, bevorzugt 0,01 bis 0,2, vorzugsweise 1 zu 0,02 bis 0,18, besonders vorzugsweise 1 zu 0,03 bis 0,15, ganz besonders vorzugsweise 1 zu 0,04 bis 0,1, insbesondere 1 zu 0,05 bis 0,06. Vorzugsweise wählt man dazu mindestens einen Alkohol aus der Reihe der C1-C10-Alkohole aus, besonders vorzugsweise mindestens einen aus der Reihe tert.-Butanol, Ethanol, Methanol, Benzylalkohol sowie Diglykolmonomethylether.

Ferner setzt man beim erfindungsgemäßen Verfahren H-Silan zu Pt vorteilhaft in einem molaren Verhältnis von 1 zu 1 x10⁻⁴ bis 1 x10⁻⁹, vorzugsweise 1 zu 1x10⁻⁵ bis 1 x10⁻⁸, insbesondere von 1 zu 1x10⁻⁵ bis 9 x10⁻⁶, ein.

Dabei verwendet man als Pt-Katalysator geeigneterweise einen Pt-Heterogenkatalysator, vorzugsweise Pt aufgebracht auf einem festen Katalysatorträger, insbesondere Pt auf Aktivkohle, oder einen Pt-Homogenkatalysator, vorzugsweise einen Pt-Komplexkatalysator, wie Hexachloroplatin(IV)säure, auch "Speier-Katalysator" genannt, insbesondere Hexachloroplatin(IV)säure gelöst in Aceton, vorzugsweise einen Pt(0)-Komplexkatalysator, besonders vorzugsweise einen "Karstedt-Katalystor", ganz besonders vorzugsweise einen Platin(0)-1,3-Divinyl-1,1,3,3-Tetramethyldisiloxan-Komplex, insbesondere einen "Karstedt-Katalysator" in Xylol oder Toluol mit einem Gehalt an Pt(0) von 0,5 bis 5 Gew.-%. Eine solche Lösung enthält in der Regel einen Platin(0) 3-Divinyl-1,1,3,3-Tetramethyldisiloxan Komplex gelöst in Xylol oder Toluol, wobei man eine erfindungsgemäß verwendete Lösung vorteilhaft in verdünnter Form einsetzt und diese vorzugsweise einen Pt-Gehalt von 0,5 bis 5 Gew.-% aufweist. So setzt man beim erfindungsgemäßen Verfahren vorteilhaft einen Pt-Katalysator aus der Reihe "Karstedt-Katalysator", insbesondere eine "Karstedt-Katalysator"-Lösung, vorzugsweise Platin(0)-1,3-Divinyl-1,1,3,3-Tetramethyldisiloxan-Komplex in Xylol oder Toluol mit einem Gehalt an Pt(0) von 0,5 bis 5 Gew.-%, Hexachloroplatin(IV)säure, vorzugsweise "Speier-Katalysator", insbesondere Hexachloroplatin(IV)säure gelöst in Aceton, oder Pt geträgert auf Aktivkohle ein.

Weiter setzt man erfindungsgemäßen Verfahren H-Silan zu Carbonsäure vorzugsweise in einem molaren Verhältnis von 1 zu 1 x10⁻³ bis 30 x10⁻³, besonders vorzugsweise 1 zu 1 x10⁻³ bis 10 x10⁻³, insbesondere von 1 zu 2 x10⁻³ bis 8 x10⁻³, ein.

Dazu wählt man die Carbonsäure bevorzugt aus der Reihe Benzoesäure, 3,5-Di-tert-butylbenzoesäure, 3,5-Di-tert-butyl-4-hydroxy-benzoesäure, Propionsäure, 2,2-Dimethylpropionsäure, Essigsäure aus.

So kann man im Allgemeinen das erfindungsgemäße Verfahren -mit all seinen Kombinationsmöglichkeiten der in der vorliegenden Beschreibung dargelegten Merkmale- wie folgt ausführen:
Für die Durchführung der erfindungsgemäßen Hydrosilylierung zur Herstellung eines Tris[3-(alkoxysilyl)propyl]isocyanurats legt man in einem Rührreaktor mit Dosier-, Heiz-/Kühl-, Rückflusssowie Destillationsvorrichtung, geeigneterweise unter Schutzgas, beispielsweise Stickstoff,
- in **Schritt A** ein Gemisch enthaltend 1,3,5-Triallyl-1,3,5-triazin-2,4,6(1H,3H,5H)-trion als Olefinkomponete, mindestens eine Carbonsäure, vorzugsweise Benzoesäure, 3,5-Di-tert-butylbenzoesäure, 3,5-Di-tert-butyl-4-hydroxy-benzoesäure, Propionsäure, 2,2-Dimethylpropionsäure und/oder Essigsäure, und einem Pt-Katalysator, geeigneterweise einem "Speier-Katalysator", vorzugsweise Hexachloroplatin(IV)säure in Aceton bzw. Hexachloroplatin(IV)säure-Hexahydrat in Aceton gelöst) oder einem "Karstedt-Katalysator", wobei dieser bevorzugt als Platin(0)-Komplexkatalysator-Lösung eingesetzt wird, oder Pt auf A-Kohle, vor, und erwärmt das Gemisch auf eine Temperatur von 40 bis 140 °C, vorzugsweise auf eine Temperatur von 50 bis 120°C,
- in **Schritt B** setzt man unter Durchmischung mindestens ein Hydrogenalkoxysilan aus der Reihe Hydrogentrialkoxysilan, Hydrogenalkyldialkoxysilan, Hydrogendialkylalkoxysilan [kurz H-Silan(e)] genannt] dem Gemisch aus Schritt A zu,
- in **Schritt C** lässt man das Gemisch aus Schritt B unter Durchmischung reagieren, wobei man dem Gemisch als weiteren Cokatalysator mindestens eine definierte Menge an Alkohol zusetzt, vorzugsweise unter Temperaturkontrolle und über 1 bis 10 oder mehr Stunden, wobei die Dosierzeit freilich von der Ansatzgröße und dem Reaktordesign abhängig sein kann, geeigneterweise kann man unter Durchmischung nachreagieren lassen, beispielsweise bei einer Temperatur von 60 bis 100 °C, insbesondere über 0,5 bis 2 Stunden, der Alkohol kann auch separat zugegeben werden,
- im anschließenden **Schritt D** arbeitet man das so erhaltene Produktgemisch auf.

So setzt man bei vorliegenden Verfahren die jeweiligen Einsatzstoffe bevorzugt einem wohl definierten molaren Verhältnis ein:
- H-Silan zu Olefinkomponente in einem molaren Verhältnis von 1 zu 0,1 bis 1
- H-Silan zu Alkohol in einem molaren Verhältnis von 1 zu 0,01 bis 0,2
- H-Silan zu Pt in einem molaren Verhältnis von 1 zu 1 x10⁻⁴ bis 1 x10⁻⁹
- H-Silan zu Carbonsäure in einem molaren Verhältnis von 1 zu 1 x10⁻³ bis 30 x10⁻³
Ferner stellt man die verwendete "Karstedt-Katalysator"-Lösung bevorzugt aus einem handelsüblichen sogenannten "Karstedt-Katalysator"Konzentrat (Platin(0) 1,3-Divinyl-1,1,3,3-Tetramethyldisiloxan Komplex, Platingehalt: 20,37 Gew.-%) (nachfolgend auch kurz "Karstedt-Konzentrat" genannt) her, wobei man das Konzentrat vorzugsweise durch den Zusatz von Xylol bzw. Toluol auf einen Pt-Gehalt von 0,5 bis 5 Gew.-% einstellt.

Ein so erhaltenes Produktgemisch arbeitet man geeigneterweise durch Destillation auf und gewinnt so das gewünschte (Ziel-)Produkt. Dazu führt man die Destillation bevorzugt beginnend bei 45 °C bis 150 °C und einem verminderten Druck (Vakuumdestillation bei kleiner 1 bar fallend, insbesondere kleiner gleich 0,1 bar) durch, wobei insbesondere vorliegende Tiefsieder, beispielsweise Carbonsäure, Alkohol, überschüssiges H-Silan bzw. ggf. noch vorliegende Olefinkomponente aus dem Produktgemisch entfernt werden. Sofern ein Pt-Heterogenkatalysator für die Durchführung des erfindungsgemäßen Verfahrens eingesetzt wird, kann man den Pt-Heterogenkatalysator im Rahmen der Produktaufarbeitung aus dem nach der Umsetzung erhaltenen Produktgemisch, d.h. vor dem Destillationsschritt, abtrennen, beispielsweise durch Filtration oder Zentrifugieren, und vorteilhaft wieder in den Prozess zurückführen.

So können erfindungsgemäß erhältliche Tris[3-(alkoxysilyl)propyl]isocyanurate in vergleichsweise hoher Ausbeute und Selektivität, d.h. mit nur geringen Anteilen an Nebenprodukten in einfacher und wirtschaftlicher Weise vorteilhaft auch großtechnisch hergestellt werden.

Die nachfolgenden Beispiele erläutern die vorliegende Erfindung zusätzlich, ohne den Gegenstand zu beschränken:

### Beispiele:

### Analytische Methoden:

### NMR-Messungen:

Instrument: Bruker
Frequenz: 500,1 MHz (¹H-NMR)
Scans: 32
Temperatur: 303 K
Lösungsmittel CDCl₃
Standard: 0,5 % TMS (Tetramethylsilan)

Nachfolgend sind Erläuterungen zur Benennung hinsichtlich Zielprodukt und bei der Synthese gebildeter Nebenprodukte bzgl. der vorliegenden ¹H-NMR-Auswertungen am Beispiel der Strukturformel eines Tris[3-(trialkoxysilyl)propyl]isocyanurats aufgeführt. Die Bestimmungen für Selektivitäten bzgl. Tris[3-(methyldialkoxysilyl)propyl]-isocyanurat und Tris[3-(dimethylalkoxysilyl)-propyl]isocyanurat wurden in analoger Weise durchgeführt und sind in den Tabellen zu den Beispielen 6 und 7 dargestellt. 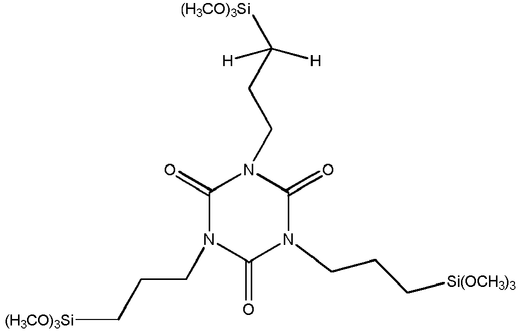 im Zielprodukt: funktionelle Gruppe S1 (Si-CH₂-) 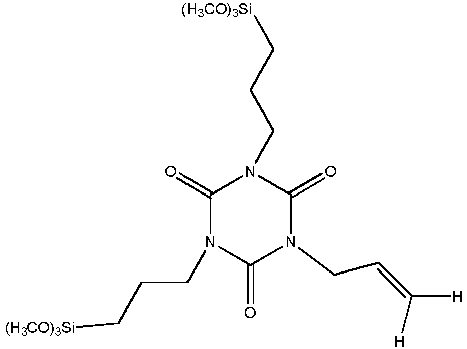 im s.g. Allylderivat: funktionelle Gruppe A1 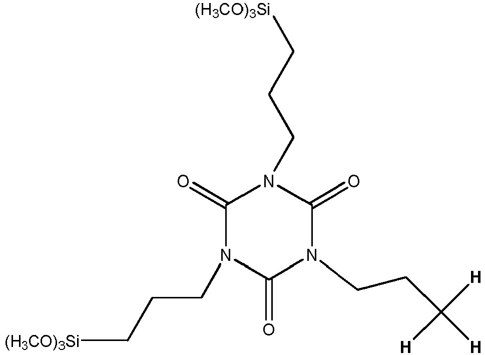 im s.g. Propylderivat: funktionelle Gruppe P1 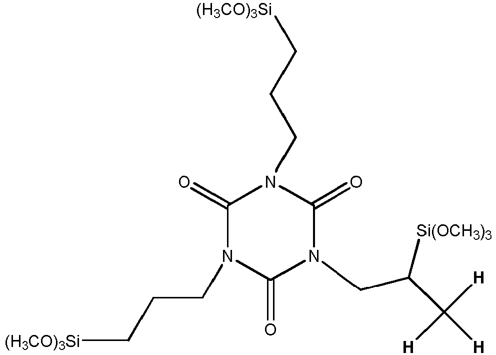 im s.g. Isopropylderivat: funtionelle Gruppe I1

Zur Auswertung der Versuche wurde das bei der Hydrosilylierung am 1,3,5-Triallyl-1,3,5-triazin-2,4,6(1H,3H,5H)-trion entstehende Produkt herangezogen. Je mehr allylische Doppelbindungen zum Zielprodukt umgesetzt und je weniger Nebenkomponenten gebildet wurden, desto besser ist die Produktqualität und die Leistungsfähigkeit/Selektivität des Katalysatorsystems. Eine hohe Selektivität ist sehr wichtig, weil die Nebenkomponenten nur mit sehr hohem Aufwand bzw. nicht vom Zielprodukt destillativ entfernt werden können.

Bei der Auswertung der 1H-NMR-Spektren wurden die in den Strukturformeln eingezeichneten Wasserstoffatome herangezogen. Bei der Hydrosilylierung entstehen Si-CH₂-Gruppen, die für das Zielprodukt charakteristisch sind. Die Si-CH₂-Gruppen wurden mit S1, die allylischen Gruppen (C=CH₂-Gruppe) mit A1, die Propylgruppe (C₃H₇-Gruppe) mit P1 und die Isopropylgruppe mit I1 gekennzeichnet. Die Auswertung der 1H-NMR-Spektren und die Berechnung der funktionellen Gruppen wurden nach jedem Versuch in Tabellen dargestellt. Die ausgewerteten Signale aus dem ¹H-NMR bilden für die Gruppe S1 und P1 Triplets (t), für die Gruppe A1 Doppel-Dublets (dd) und für die Gruppe I1 Dublets (d).

### Verwendete Chemikalien:

"Karstedt-Konzentrat" (Platin(0) 1,3-Divinyl-1,1,3,3-Tetramethyldisiloxan Komplex, Platingehalt: 20,37 Gew.-%), HERAEUS
Aceton, rein, LABC Labortechnik
Hexachloroplatin(IV)säure-hexahydrat, Platingehalt 40 Gew.-%, HERAEUS
Platin-Aktivkohle, Hydrierkatalysator, Platingehalt 10 Gew.-%, MERCK
Benzylalkohol, puriss, SIGMA ALDRICH
Diethylenglykolmonomethylether > 98 Gew.-%, MERCK
Xylol Technical, VWR Chemicals
Dynasylan® TMOS (Trimethoxysilan), EVONIK Industries
Dynasylan® TEOS-H (Triethoxysilan), EVONIK Industries
Dynasylan® DEMS (Methyldiethoxysilan), EVONIK Industries
Dynasylan® DMES (Dimethylethoxysilan), EVONIK Industries
TIACROS® (1,3,5-Triallyl-1,3,5-triazin-2,4,6(1H,3H,5H)-trion), EVONIK-Industries
Benzoesäure ≥ 99,5 Gew.-%, ROTH
3,5-Di-tert-butylbenzoesäure >98,0 Gew.-%, TOKYO CHEMICAL INDUSTRY
3,5-Di-tert-butyl-4-hydroxybenzoesäure, > Gew.-98,0 %, TOKYO CHEMICAL INDUSTRY
Essigsäure, ≥ 99 Gew.-%, SIGMA-ALDRICH
Methanol ≥ 99,5 Gew.-%, MERCK
Ethanol ≥ 99,8 Gew.-%, ROTH
tert.-Butanol, ≥ 99,0 Gew.-% (zur Synthese), ROTH
Chloroform-d1 (CDCl₃) + 0,5 Gew.-% TMS, DEUTERO
Benzol-d6, DEUTERO
Tetramethylsilan, DEUTERO

### Herstellung "Karstedt-Katalysator" Nr.1 mit 2 Gew.-% Platin Gehalt in Xylol:

In einer 0,2 l Glasflasche wurden 9,8 g "Karstedt Konzentrat" (Platin(0) 1,3-Divinyl-1,1,3,3-Tetramethyldisiloxan Komplex, Platin Gehalt 20,37 %) mit 90,2 g Xylol vermischt.

### Herstellung "Karstedt-Katalysator" Nr. 2 mit 2 Gew.-% Platin Gehalt in Toluol:

In einer 0,2 l Glasflasche wurden 9,8 g "Karstedt Konzentrat" (Platin(0) 1,3-Divinyl-1,1,3,3-Tetramethyldisiloxan Komplex, Platin Gehalt 20,37 Gew.-%) mit 90,2 g Toluol vermischt.

### Herstellung "Karstedt-Katalysator" Nr. 3 mit 0,4 Gew.-% Platin Gehalt:

In einer 0,1 l Glasflasche wurden 196,4 mg "Karstedt Konzentrat" (Platin(0) 1,3-Divinyl-1,1,3,3-Tetramethyldisiloxan Komplex, Platin Gehalt 20,37 Gew.-%) mit 9,8 g Toluol vermischt.

### Herstellung Katalysator 4 aus Hexachloroplatin(IV)säurehexahydrat Lösung in Aceton mit 2,34 Gew.-% Pt Gehalt:

In einem 12 l Kunststoffbehälter wurden 530 g H₂PtCl₆x6H₂O in 9,8 l Aceton aufgelöst. Die so hergestellte Katalysatorlösung wurde nach einer Reifung von 8 Wochen eingesetzt.

### Anmerkung zu den nachfolgenden Vergleichsbeispielen:

Die in US 5,986,124 beschriebene Synthese in Ampullen kann nicht im technischen Maßstab durchgeführt werden. Damit die Versuche besser mit den erfindungsgemäßen Beispielen verglichen werden können, wurden die Versuche in einem Rührkessel bzw. Kolben durchgeführt. Darüber hinaus wurden bei den Beispielen in US 5,986,124 andere ungesättigte Verbindungen eingesetzt, sodass ein direkter Vergleich mit der vorliegenden Erfindung nicht möglich wäre; somit wurde in den folgenden Vergleichsbeispielen TAICROS ® eingesetzt.

### Vergleichsbeispiel 1: (in Anlehnung an Beispiel 1 aus US 5,986,124)

0,2003 Mol (24,5 g) Dynasylan® TMOS, 0,1 ml Katalysator Nr.1, weitere 40,0 g Toluol als zusätzliches Löse- bzw. Verdünnungsmittel, 0,0665 Mol (16,6 g) TAICROS® und 0,4 ml Essigsäure wurden in einer 0,25 I-Rührapparatur mit Intensivkühler vorgelegt und 2,5 Stunden in einem auf 53 - 55 °C erwärmten Ölbad gerührt. Dabei wurden 79,9 g unvollständig umgesetztes und farbloses Sumpfprodukt erhalten. Die leicht flüchtigen Komponenten wurden nicht entfernt.

Auswertung des ¹H-NMR Spektrums bzgl. Vergleichsbeispiel 1:

| Lösungsmittel: CDCl₃ + 0,5 % TMS | Signal bei [ppm] | Integral I | N Anzahl der Protonen | I / N | % (Mol) |
|---|---|---|---|---|---|
| S1 | 0,64 | 100,00 | 2 | 50,00 | 45,8 |
| A1 | 5,27 | 117,28 | 2 | 58,64 | 53,7 |
| P1 | 0,93 | 0,81 | 3 | 0,27 | 0,3 |
| I1 | 1,00 | 0,61 | 3 | 0,20 | 0,2 |

Ergebnis: 45,8 % der Allylgruppen wurden durch Hydrosilylierung in Trimethoxysilyalkylgruppen (vgl. S1) umgewandelt. 53,7 % der Allylgruppen (A1) sind nicht umgesetzt worden und es entstanden 0,3 % Propyl (P1)- bzw. 0,2 % iso-Propylgruppen (I1), die das Produkt verunreinigen.
Die Reaktion ist nicht vollständig.

### Vergleichsbeispiel 2: (in Anlehnung an Beispiel 1 aus US 5,986,124)

0,2003 Mol (32,9 g) Dynasylan® TEOS-H, 0,1 ml Katalysator Nr. 3, weitere 40,0 g Toluol als zusätzliches Löse- bzw. Verdünnungsmittel, 0,0665 Mol (16,6 g) TAICROS® und 0,4 ml Essigsäure wurden in einer 0,25 I-Rührapparatur mit Rückflusskühler, vorgelegt und 2,5 Stunden in einem auf 50 - 57 °C erwärmten Ölbad gerührt. Dabei wurden 88,2 g unvollständig umgesetztes und farbloses Sumpfprodukt erhalten. Die leicht flüchtigen Komponenten wurden nicht entfernt.

Auswertung des ¹H-NMR-Spektrums bzgl. Vergleichsbeispiel 2:

| Lösungsmittel: CDCl₃ + 0,5 % TMS | Signal bei [ppm] | Integral I | N Anzahl der Protonen | I / N | % (Mol) |
|---|---|---|---|---|---|
| S1 | 0,64 | 100,00 | 2 | 50,00 | 86,2 |
| A1 | 5,26 | 14,59 | 2 | 7,30 | 12,6 |
| P1 | 0,94 | 0,33 | 3 | 0,33 | 0,6 |
| I1 | 1,06 | 0,35 | 3 | 0,35 | 0,6 |

Ergebnis: 86,2 % der Allylgruppen wurden durch Hydrosilylierung in Trimethoxysilyalkylgruppen (vgl. S1) umgewandelt. 12,6 % der Allylgruppen (A1) sind nicht umgesetzt worden und es entstanden 0,6 % Propyl (P1)- bzw. 0,6 % iso-Propylgruppen (I1), die das Produkt verunreinigen.
Die Reaktion ist nicht vollständig.

### Vergleichsbeispiel 3: (nur mit Essigsäure, ohne Alkoholzusatz)

1,2 Mol DYNASYLAN® TMOS und 0,2 g Katalysator Nr. 1 (entspricht 0,0205 mMol Pt) in einer 0,5 I-Rührapparatur mit Rückflusskühler, Dosiervorrichtung vorgelegt. Bei einer Temperatur von 76 - 91 °C wurde eine Mischung bestehend aus 0,33 Mol TAICROS® und 6,77 mMol Essigsäure innerhalb 1 h dosiert. Danach ließ man das Gemisch noch ca. 1 weitere Stunde bei ca. 87 - 92 °C weiterreagieren. Anschließend wurden 55,0 g Tiefsieder bei 90 - 120 °C und einem Druck von < 0,1 mbar entfernt. Dabei wurden 170,7 g unvollständig umgesetztes farbloses Sumpfprodukt erhalten.

Auswertung des ¹H-NMR-Spektrums bzgl. Vergleichsbeispiel 3:

| Lösungsmittel: CDCl₃ + 0,5 % TMS | Signal bei [ppm] | Integral I | N Anzahl der Protonen | I / N | % (Mol) |
|---|---|---|---|---|---|
| S1 | 0,66 | 100,00 | 2 | 50,00 | 73,8 |
| A1 | 5,25 | 34,79 | 2 | 17,40 | 25,7 |
| P1 | 0,94 | 0,61 | 3 | 0,20 | 0,3 |
| I1 | 1,01 | 0,43 | 3 | 0,14 | 0,2 |

Ergebnis: 73,8 % der Allylgruppen wurden durch Hydrosilylierung in Trimethoxysilyalkyllgruppen (vgl. S1) umgewandelt. 25,7 % der Allylgruppen (A1) sind nicht umgesetzt worden und es entstanden 0,3 % Propyl (P1)- bzw. 0,2 % iso-Propylgruppen (I1), die das Produkt verunreinigen.
Die Reaktion ist nicht vollständig.

### Vergleichsbeispiel 4:

1,2 Mol Dynasylan® TMOS, 0,2 g "Karstedt-Katalysator" (entspricht 0,0205 mMol Pt), 34,38 mMol Methanol und 6,55 mMol Benzoesäure wurden in einer 0,5 I-Rührapparatur mit Rückflusskühler, Dosiervorrichtung vorgelegt. Bei einer Temperatur von 73 - 82 °C wurden 0,33 Mol TAICROS® innerhalb 1 h Stunde dosiert. Danach ließ man das Gemisch noch 1 Stunde bei 81 °C weiterreagieren. Anschließend wurden 89,5 g Tiefsieder bei 35 - 127 °C und einem Druck von < 0,1 mbar entfernt. Dabei wurde 134,2 g nicht vollständig umgesetztes und farbloses Sumpfprodukt erhalten.

Auswertung des ¹H-NMR-Spektrums aus Vergleichsbeispiel 4:

| Lösungsmittel: CDCl3 + 0,5 % TMS | Signal bei [ppm] | Integral | N Anzahl der Protonen | I / N | % (Mol) |
|---|---|---|---|---|---|
| S1 | 0,66 | 100,00 | 2 | 50,00 | 42,5 |
| A1 | 5,25 | 134,10 | 2 | 67,05 | 57,0 |
| P1 | 0,94 | 1,24 | 3 | 0,41 | 0,4 |
| I1 | 1,01 | 0,36 | 3 | 0,12 | 0,1 |

Ergebnis: 42,5 % der Allylgruppen wurden durch Hydrosilylierung mit TMOS in Trimethoxysilylalkylgruppen (vgl. S1) umgewandelt. 57,0 % der Allylgruppen (A1) wurden nicht umgesetzt. Es entstanden 0,4 % Propyl (P1)- bzw. 0,1 % iso-Propylgruppen (I1), die das Produkt verunreinigen. Die Umsetzung der Allylgruppen ist nicht vollständig und es entstanden nur wenige Nebenprodukte

### Vergleichsbeispiel 5:

1,2 Mol Dynasylan® TMOS, 0,2 g "Karstedt-Katalysator" (entspricht 0,0205 mMol Pt) und 34,38 mMol Methanol wurden in einer 0,5 l-Rührapparatur mit Rückflusskühler, Dosiervorrichtung vorgelegt. Bei einer Temperatur von 70 - 87 °C wurde eine Mischung bestehend aus 0,33 Mol TAICROS® und 6,55 mMol Benzoesäure innerhalb 1 h Stunde dosiert. Danach ließ man das Gemisch noch 1 Stunde bei 81 °C weiterreagieren. Anschließend wurden 41,5 g Tiefsieder bei 61 - 121 °C und einem Druck von < 0,1 mbar entfernt. Dabei wurde 183,0 g nicht vollständig umgesetztes und farbloses Sumpfprodukt erhalten.

| Auswertung des 1H-NMR-Spektrums Vergleichsbeispi el 5:Lösungsmittel: CDCl3 + 0,5 % TMS | Signal bei [ppm] | Integral | N Anzahl der Protonen | I / N | % (Mol) |
|---|---|---|---|---|---|
| S1 | 0,66 | 100,0 | 2 | 50,00 | 81,5 |
| A1 | 5,25 | 21,75 | 2 | 10,87 | 17,7 |
| P1 | 0,94 | 1,09 | 3 | 0,36 | 0,6 |
| I1 | 1,01 | 0,38 | 3 | 0,13 | 0,2 |

Ergebnis: 81,5 % der Allylgruppen wurden durch Hydrosilylierung mit TMOS in Trimethoxysilylalkylgruppen (vgl. S1) umgewandelt. 17,7 % der Allylgruppen (A1) wurden nicht umgesetzt. Es entstanden 0,6 % Propyl (P1)- bzw. 0,2 % iso-Propylgruppen (I1), die das Produkt verunreinigen. Die Umsetzung der Allylgruppen ist nicht vollständig und es entstanden nur wenige Nebenprodukte

### Vergleichsbeispiel 6:

0,33 Mol (83,1 g) TAICROS®, 0,2 g Katalysator Nr. 1 (entspricht 0,0205 mMol Pt) und 6,79 mMol (1,7 g) 3,5-Di-tert-butyl-4-hydroxy-benzoesäure wurden in einer 0,5 I-Rührapparatur mit Rückflusskühler, Dosiervorrichtung vorgelegt. Bei einer Temperatur von 91 - 111 °C sollten 1,2 Mol (146,6 g) Dynasylan® TMOS dosiert werden. Die Hydrosilylierung ist stark exotherm und nach einer Dosierung von 18 g Dynasylan® TMOS innerhalb von 9 Minuten stieg die Temperatur bereits von 91 auf 97 °C an. Nachdem weitere 72 g Dynasylan® TMOS innerhalb von 27 Minuten dosiert waren und die Temperatur auf 108 °C angestiegen war, konnte bei weiterer Zugabe von Dynasylan® TMOS keine Exothermie festgestellt werden. Das Reaktionsgemisch kühlte innerhalb von wenigen Minuten von 108 auf 89 °C ab. Der Versuch wurde deshalb nach einer Dosierung von insgesamt 90 g Dynasylan® TMOS abgebrochen, d.h. die Reaktion kam zum Stillstand und die Umsetzung blieb bei dieser Verfahrensweise somit entsprechend unvollständig. 68 g Dynasylan® TMOS wurden nicht dosiert.

### Anmerkung:

Die vorliegenden Vergleichsversuche zur Herstellung von Tris[3-(alkoxysilyl)propyl]isocyanuraten durch Hydrosilylierung von 1,3,5-Triallyl-1,3,5-triazin-2,4,6(1H,3H,5H)-trion (TAICROS®) in Gegenwart eines Pt-Katalysatorsystems aus Pt-Katalysator und Carbonsäure zeigen, dass man einen vergleichsweise geringer Umsatz der Doppelbindung von deutlich unterhalb 90 Mol-% auffindet, wenn man
- ein Gemisch aus H-Silan, Pt-Katalysator, Carbonsäure und TAICROS® einsetzt, erwärmt und so zur Reaktion bringt
- H-Silan und Pt-Katalysator vorlegt, erwärmt und eine Gemisch aus TAICROS® und Carbonsäure zudosiert
- H-Silan, Pt-Katalysator und Alkohol vorlegt, erwärmt und eine Gemisch aus TAICROS® und Carbonsäure zudosiert
- H-Silan, Pt-Katalysator, Carbonsäure und Alkohol vorlegt, erwärmt und TAICROS® zudosiert oder
- TAICROS®, Pt-Katalysator und Carbonsäure vorlegt, erwärmt und H-Silan zudosiert.

### Beispiel 1:

0,33 Mol TAICROS®, 0,2 g Katalysator Nr. 1 (entspricht 0,0205 mMol Pt) und 6,79 mMol 3,5-Di-tert-butyl-4-hydroxy-benzoesäure wurden in einer 0,5 I-Rührapparatur mit Rückflußkühler, Dosiervorrichtung vorgelegt. Bei einer Temperatur von 91-111°C wurden 0,82 Mol Dynasylan® TMOS innerhalb von 45 Minuten dosiert. Anschließend wurden 33,7 mMol Tert-butanol zugegeben und bei einer Temperatur von 96-111°C 0,38 Mol Dynasylan® TMOS innerhalb von 20 Minuten dosiert. Danach ließ man das Gemisch noch ca. 1 weitere Stunde bei ca. 109-120°C weiterreagieren. Anschließend wurden 24,3 g Tiefsieder bei ca. 104-120°C und einem Druck von < 0,1 mbar entfernt. Dabei wurde 204,9 g vollständig umgesetztes und farbloses Sumpfprodukt erhalten. Neben dem Zielprodukt wurden folgende Spurenverunreinigungen über die ¹H-NMR-Spektren ausgewertet:

### Auswertung des ¹H-NMR-Spektrums.

| Lösungsmittel: CDCl₃ + 0,5 % TMS | Signal bei [ppm] | Integral | N Anzahl der Protonen | I / N | % (Mol) |
|---|---|---|---|---|---|
| S1 | 0,66 | 100,0 | 2 | 50,00 | 97,3 |
| A1 | 5,25 | 0,02 | 2 | 0,01 | < 0,1 |
| P1 | 0,94 | 2,09 | 3 | 0,70 | 1,4 |
| I1 | 1,01 | 2,01 | 3 | 0,67 | 1,3 |

Ergebnis: 97,3% der Allylgruppen wurden durch Hydrosilylierung mit TMOS in Trimethoxysilylgruppen umgewandelt. Allylgruppen (A1) sind noch in sehr geringen Mengen nachweisbar. Es entstanden 1,4 % Propyl (P1)- bzw. 1,3 % iso-Propylgruppen (I1), die das Produkt verunreinigen. Die Umsetzung der Allylgruppen ist vollständig und es entstanden nur wenige Nebenprodukte.

## Patentansprüche

1. Verfahren zur Herstellung eines Tris[3-(alkoxysilyl)propyl]isocyanurats aus der Reihe Tris[3-(Trialkoxysilyl)propyl]isocyanurat, Tris[3-(Alkyldialkoxysilyl)propyl]isocyanurat und Tris[3-(Dialkylalkoxysilyl)propyl]isocyanurat durch Hydrosilylierung,
indem man
- in Schritt A ein Gemisch enthaltend 1,3,5-Triallyl-1,3,5-triazin-2,4,6(1H,3H,5H)-trion als Olefinkomponete, mindestens eine Carbonsäure und einen Pt-Katalysator vorlegt, das Gemisch auf eine Temperatur von 40 bis 140 °C erwärmt,
- in Schritt B unter Durchmischung mindestens ein Hydrogenalkoxysilan aus der Reihe Hydrogentrialkoxysilan, Hydrogenalkyldialkoxysilan, Hydrogendialkylalkoxysilan [kurz H-Silan(e)] genannt] dem Gemisch aus Schritt A zusetzt,
- in Schritt C das Gemisch aus Schritt B unter Durchmischung reagieren lässt, wobei man dem Gemisch als weiteren Cokatalysator mindestens eine definierte Menge an Alkohol zusetzt und
- in Schritt D das so erhaltene Produktgemisch aufarbeitet.

2. Verfahren nach Anspruch 1, **dadurch gekennzeichnet,**
**dass** man H-Silan zu Alkohol in einem molaren Verhältnis von 1 zu 0,005 bis 0,3, bevorzugt 0,01 bis 0,2, vorzugsweise 1 zu 0,02 bis 0,18, besonders vorzugsweise 1 zu 0,03 bis 0,15, ganz besonders vorzugsweise 1 zu 0,04 bis 0,1, insbesondere 1 zu 0,05 bis 0,06, einsetzt.

3. Verfahren nach Anspruch 1 oder 2, **dadurch gekennzeichnet,**
**dass** man H-Silan zu Pt in einem molaren Verhältnis von 1 zu 1 x10⁻⁴ bis 1 x10⁻⁹, vorzugsweise 1 zu 1x10⁻⁵ bis 3 x10⁻⁸, insbesondere 1 zu 1x10⁻⁵ bis 9,0 x10⁻⁶, einsetzt.

4. Verfahren nach einem der vorangehenden Ansprüchen, **dadurch gekennzeichnet,**
**dass** man H-Silan zu Carbonsäure in einem molaren Verhältnis von 1 zu 1x10⁻³ bis 30x10⁻³, vorzugsweise 1 zu 2x10⁻³ bis 8x10⁻³, einsetzt.

5. Verfahren nach einem der vorangehenden Ansprüchen, **dadurch gekennzeichnet,**
**dass** man H-Silan zu Olefinkomponente in einem molaren Verhältnis von 1 zu 0,1 bis 1, vorzugsweise 1 zu 0,2 bis 0,4, einsetzt.

6. Verfahren nach einem der vorangehenden Ansprüchen, **dadurch gekennzeichnet,**
**dass** man die Carbonsäure aus der Reihe Benzoesäure, Propionsäure, 2,2-Dimethylpropionsäure, 3,5-Di-tert-butylbenzoesäure, 3,5-Di-tert-butyl-4-hydroxybenzoesäure, Essigsäure auswählt.

7. Verfahren nach einem der vorangehenden Ansprüchen, **dadurch gekennzeichnet,**
**dass** man den Alkohol aus der Reihe der C1-C10-Alkohole auswählt, vorzugsweise aus der Reihe tert.-Butanol, Ethanol, Methanol, Benzylalkohol sowie Diglykolmonomethylether.

8. Verfahren nach einem der vorangehenden Ansprüchen, **dadurch gekennzeichnet,**
**dass** man als H-Silan Hydrogentrimethoxysilan (TMOS), Hydrogentriethoxysilan (TEOS), Methyldiethoxysilan (DEMS), Methyldimethoxysilan (DMMS), Dimethylethoxysilan (DMES) oder Dimethylmethoxysilan (MDMS) einsetzt.

9. Verfahren nach einem der vorangehenden Ansprüchen, **dadurch gekennzeichnet,**
**dass** man einen Pt-Katalysator aus der Reihe "Karstedt-Katalysator", vorzugsweise Platin(0)-1,3-Divinyl-1,1,3,3-Tetramethyldisiloxan-Komplex, insbesondere als "Karstedt-Katalysator" in Xylol oder Toluol mit einem Gehalt an Pt(0) von 0,5 bis 5 Gew.-%, Hexachloroplatin(IV)säure, vorzugsweise "Speier-Katalysator", insbesondere Hexachloroplatin(IV)säure gelöst in Aceton, oder Pt aufgebracht auf einem festen Katalysatorträger, vorzugsweise Pt geträgert auf Aktivkohle, einsetzt.

## Claims

1. Process for preparing a tris[3-(alkoxysilyl)propyl] isocyanurate from the group of tris[3-(trialkoxysilyl)propyl] isocyanurate, tris[3-(alkyldialkoxysilyl)propyl] isocyanurate and tris[3-(dialkylalkoxysilyl)propyl] isocyanurate by hydrosilylation,
by
- in step A initially charging a mixture comprising 1,3,5-triallyl-1,3,5-triazine-2,4,6(1H,3H,5H)-trione as olefin component, at least one carboxylic acid and a Pt catalyst, heating the mixture to a temperature of 40 to 140°C,
- in step B adding at least one hydroalkoxysilane from the group of hydrotrialkoxysilane, hydroalkyldialkoxysilane, hydrodialkylalkoxysilane [called H-silane(s) for short] to the mixture from step A while mixing,
- in step C leaving the mixture from step B to react while mixing, with addition of at least a defined amount of alcohol to the mixture as further cocatalyst, and
- in step D working up the product mixture thus obtained.

2. Process according to Claim 1, **characterized in that**
H-silane is used relative to alcohol in a molar ratio of 1:0.005 to 0.3, preferably 0.01 to 0.2,
preferably 1:0.02 to 0.18, more preferably 1:0.03 to 0.15, even more preferably 1:0.04 to 0.1, especially 1:0.05 to 0.06.

3. Process according to Claim 1 or 2, **characterized in that**
H-silane is used relative to Pt in a molar ratio of 1:1 x 10⁻⁴ to 1 x 10⁻⁹, preferably 1:1 x 10⁻⁵ to 3 x 10⁻⁸, especially 1:1 x 10⁻⁵ to 9.0 x 10⁻⁶.

4. Process according to any of the preceding claims, **characterized in that**
H-silane is used relative to carboxylic acid in a molar ratio of 1:1 x 10⁻³ to 30 x 10⁻³, preferably 1:2 x 10⁻³ to 8 x 10⁻³.

5. Process according to any of the preceding claims, **characterized in that**
H-silane is used relative to olefin component in a molar ratio of 1:0.1 to 1, preferably 1:0.2 to 0.4.

6. Process according to any of the preceding claims, **characterized in that**
the carboxylic acid is selected from the group of benzoic acid, propionic acid, 2,2-dimethylpropionic acid, 3,5-di-tert-butylbenzoic acid, 3,5-di-tert-butyl-4-hydroxybenzoic acid, acetic acid.

7. Process according to any of the preceding claims, **characterized in that**
the alcohol is selected from the group of the C1-C10 alcohols, preferably from the group of tert-butanol, ethanol, methanol, benzyl alcohol and diglycol monomethyl ether.

8. Process according to any of the preceding claims, **characterized in that**
the H-silane used is hydrotrimethoxysilane (TMOS), hydrotriethoxysilane (TEOS), methyldiethoxysilane (DEMS), methyldimethoxysilane (DMMS), dimethylethoxysilane (DMES) or dimethylmethoxysilane (MDMS).

9. Process according to any of the preceding claims, **characterized in that**
a Pt catalyst from the "Karstedt catalyst" group is used, preferably platinum(0)-1,3-divinyl-1,1,3,3-tetramethyldisiloxane complex, especially in the form of "Karstedt catalyst" in xylene or toluene with a Pt(0) content of 0.5% to 5% by weight, hexachloroplatinum(IV) acid, preferably "Speier catalyst", especially hexachloroplatinum(IV) acid dissolved in acetone, or Pt applied to a solid catalyst support, preferably Pt supported on activated carbon.

## Revendications

1. Procédé pour la préparation d'un isocyanurate de tris[3-(alcoxysilyl)propyle] de la série de l'isocyanurate de tris[3-(trialcoxysilyl)propyle], de l'isocyanurate de tris[3-(alkyldialcoxysilyl)propyle] et de l'isocyanurate de tris[3-(dialkylalcoxysilyl)propyle] par hydrosilylation,
dans lequel
- dans une étape A, on fournit un mélange contenant de la 1,3,5-triallyl-1,3,5-triazine-2,4,6(1H,3H,5H)-trione en tant que composant d'oléfine, au moins un acide carboxylique et un catalyseur au Pt, on chauffe le mélange à une température de 40 à 140 °C,
- dans une étape B, on ajoute au mélange de l'étape A, sous agitation, au moins un hydrogénoalcoxysilane de la série hydrogénotrialcoxysilane, hydrogénoalkyldialcoxysilane, hydrogénodialkylalcoxysilane [en abrégé H-silane(s)],
- dans une étape C, on laisse réagir le mélange de l'étape B sous agitation, dans lequel on ajoute au mélange, en tant que co-catalyseur supplémentaire, au moins une quantité définie d'alcool et
- dans une étape D, on traite le mélange de produits ainsi obtenu.

2. Procédé selon la revendication 1, **caractérisé en ce qu'**on utilise le H-silane par rapport à l'alcool en un rapport molaire de 1 sur 0,005 à 0,3, préférablement 0,01 à 0,2, de préférence de 1 sur 0,02 à 0,18, particulièrement préférablement de 1 sur 0,03 à 0,15, tout particulièrement préférablement de 1 sur 0,04 à 0,1, en particulier de 1 sur 0,05 à 0,06.

3. Procédé selon la revendication 1 ou 2, **caractérisé en ce qu'**on utilise le H-silane par rapport au Pt en un rapport molaire de 1 sur 1 x 10⁻⁴ à 1 x 10⁻⁹, de préférence de 1 sur 1 x 10⁻⁵ à 3 x 10⁻⁸, en particulier de 1 sur 1 x 10⁻⁵ à 9,0 x 10⁻⁶.

4. Procédé selon l'une quelconque des revendications précédentes, **caractérisé en ce qu'**on utilise le H-silane par rapport à l'acide carboxylique en un rapport molaire de 1 sur 1 x 10⁻³ à 30 x 10⁻³, de préférence de 1 sur 2 x 10⁻³ à 8 x 10⁻³.

5. Procédé selon l'une quelconque des revendications précédentes, **caractérisé en ce qu'**on utilise le H-silane par rapport au composant d'oléfine en un rapport molaire de 1 sur 0,1 à 1, de préférence de 1 sur 0,2 à 0,4.

6. Procédé selon l'une quelconque des revendications précédentes, **caractérisé en ce qu'**on choisit l'acide carboxylique dans la série acide benzoïque, acide propionique, acide 2,2-diméthylpropionique, acide 3,5-di-tert-butylbenzoïque, acide 3,5-di-tert-butyl-4-hydroxybenzoïque, acide acétique.

7. Procédé selon l'une quelconque des revendications précédentes, **caractérisé en ce qu'**on choisit l'alcool dans la série des alcools en C₁₋₁₀, de préférence dans la série tert-butanol, éthanol, méthanol, alcool benzylique ainsi que monométhyléther de diglycol.

8. Procédé selon l'une quelconque des revendications précédentes, **caractérisé en ce qu'**en tant que H-silane, on utilise de l'hydrogénotriméthoxysilane (TMOS), de l'hydrogénotriéthoxysilane (TEOS), du méthylediéthoxysilane (DEMS), du méthylediméthoxysilane (DMMS), du diméthyléthoxysilane (DMES) ou du diméthylméthoxysilane (MDMS).

9. Procédé selon l'une quelconque des revendications précédentes, **caractérisé en ce qu'**on utilise un catalyseur au Pt de la série « catalyseur de Karstedt », de préférence un complexe platine(0)-1,3-divinyl-1,1,3,3-tétraméthyldisiloxane, en particulier en tant que « catalyseur de Karstedt » dans le xylène ou le toluène avec une teneur en Pt(0) de 0,5 à 5 % en poids, l'acide hexachloroplatinique(IV), de préférence « le catalyseur de Speier », en particulier l'acide hexachloroplatinique(IV) dissous dans l'acétone, ou Pt déposé sur un support solide de catalyseur, de préférence Pt supporté sur du charbon actif.
